(19) Europäisches Patentamt
European Patent Office
Office européen des brevets

(11) Publication number: **0 139 526**
**A2**

(12) # EUROPEAN PATENT APPLICATION

(21) Application number: **84307169.7**

(22) Date of filing: **18.10.84**

(51) Int. Cl.$^4$: **G 01 N 33/543**
G 01 N 33/531

(30) Priority: **20.10.83 US 543724**

(43) Date of publication of application:
**02.05.85 Bulletin 85/18**

(84) Designated Contracting States:
**BE CH DE FR GB LI NL SE**

(71) Applicant: **WARNER-LAMBERT COMPANY**
**201 Tabor Road**
**Morris Plains New Jersey 07950(US)**

(72) Inventor: **Barnes, Wayne R.**
**18 788 Linden Avenue**
**Gray Lakes Illinois 60048(US)**

(72) Inventor: **Guzzaldo, Carole**
**121 Simmons**
**Loppell Texas 75019(US)**

(72) Inventor: **Morrison, Julie D.**
**14100 Montfort Drive**
**Dallas Texas 75290(US)**

(74) Representative: **Jones, Michael Raymond et al,**
**HASELTINE LAKE & CO. Hazlitt House 28 Southampton Buildings Chancery Lane**
**London WC2A 1AT(GB)**

(54) **An insolubilized stable specific binding protein.**

(57) There is disclosed an insolubilized stable specific binding protein which is prepared by reacting a specific binding protein with an aldehyde compound and a reducing agent. A process for preparing a solid phase immunoassay and a kit comprising the insolubilized stable specific binding protein are also disclosed.

EP 0 139 526 A2

AN INSOLUBILIZED STABLE SPECIFIC BINDING PROTEIN

The present invention relates to a insolubilized stable specific binding protein and method for preparing a solid phase immunoassay and more particularly to an aldehyde protein conjugate bound in the presence of a reducing agent to an insoluble support and its use as a solid phase immunoassay.

Many methods have been proposed for preparing solid phase immunoassays. These methods have used both active and passive adsorption techniques. One of the passive techniques is disclosed by Catt, in U.S. Patent 3,646,346 wherein the inner surface of a water-insoluble material, such as a test tube, is contacted with a protein whereby the surface is coated by passive adsorption. The coated tube is then brought into contact with an aqueous sample containing the material to be assayed in the presence of a known quantity of labeled material. While this process was originally successful, a disadvantage typically found with passively coated protein is susceptibility to desorption during the assay incubation resulting in inconsistent results.

Such difficulties associated with passive adsorption have attempted to be overcome with active absorption techniques. These latter techniques have primarily involved the use of covalent bonding agents to couple the protein to the solid support. One such technique is disclosed by Barrett in U.S. Patent 4,001,583 wherein biological substances are covalently bonded to plastic material whose inside surfaces have been previously coated with glutaraldehyde, with or without prior treatment with an aliphatic amine or diamine. Barrett asserts that the coupling effect is achieved by the self-polymerization of the aldehyde material on the surface of the plastic followed by a Schiff base-type coupling of the protein to the active unreacted aldehyde group.

Another process is described in U.S. Patent 4,069,352 to Parsons for performing an active absorption technique. Parsons discloses a solid phase immunoadsorbent prepared by crosslinking a low titre antiserum to form a protein structure having an increased molecular weight. In general the extent of crosslinking desired for optimum results is from two to five times the molecular weight of the uncrosslinked starting material; that is the uncrosslinked antibody or binding protein. This crosslinked low titre material is then absorbed onto the surface of a water insoluble polymeric material such as a test tube. The absorption reaction is performed at a pH of 8 to 10. Parsons discloses performing the crosslinking procedure with a bifunctional reagent, such as glutaraldehyde before bringing the antiserum into contact with the surface of the polymeric material at this critical pH.

While active adsorption techniques have increased binding capability, such methods when using aldehyde coupling agents have not always provided sufficient binding enhancement to benefit immunoassay effectiveness.

It has been unexpectedly discovered that certain specific binding proteins which show little apparent immunoreactivity when activated with mono- or bifunctional aldehydes can successfully be employed in immunoassays if activated with an aldehyde and coated on the solid phase in the presence of a reducing agent.

More particularly, the invention involves an insolublized, stable specific binding protein and a method for preparing a solid phase immunoassay using specific binding protein reacted with an aldehyde and incubated in the presence of a reducing agent. When the aldehyde/reducing agent is in combination with a specific binding protein, significant enhancement in immunoreactive stability of the coating solution and/or improvement in the percent bound fraction of the labeled analyte is observed.

As used herein, the term "specific binding protein" means a group of serum proteins, also referred to as gamma globulins or immunoglobulins, that will specifically react with an antibody, antigen or hapten. Most of the specific binding proteins belong to the immunoglobulin subclass known as IgG, while other sub-classes are referred to as IgA, IgM, IgD and IgE. This term is also used to include certain binding proteins which recognize certain antigens, such as thyroxine binding globulin for thyroxine. While not being limited thereto, the invention has been found to be quite effective with low molecular weight substances, for example, steroids, vitamin $B_{12}$, thyroxine and triiodothyronine, releasing factors, histamine, serotonin and other biogenic amines, digoxine, digitoxin, prostanglandins, adrenalin, nor-adrenalin, vegatable hormones such as auxin, kinetin and gibberellic acid.

The term "kit" as employed herein means a collection of all or some of the chemicals, including the assay tubes, and instructions necessary to do an immunoassay.

It has been found that specific binding proteins which are prevented from successful development into suitable immunoassays are rendered usable according to the present invention by the addition of a reducing agent to the coating solution both prior to and after the aldehyde activation step has been completed. When either the aldehyde compound or reducing agent are employed alone with the specific binding protein in the absence of the other agent, the resulting binding efficiency is less than that achieved when using both of these materials. The presence of both of these reagents is essential to achieve a synergestic effect by preventing loss of antibody inhibitory activity.

In the Drawings,

Figure 1 depicts the amount of bound analyte in the presence and absence of 1,4-dithiothreitol of Example 1.

Figure II depicts the amount of bound analyte in the presence and absence of 1,4-dithiothreitol of Example 2.

The specific binding protein used in the inventive process may be any purified or non-purified antiserum. Preferably the specific binding protein is purified to enhance the specific immunoglobulin efficiency of the specific binding proteins active constituents when bonded to the insoluble support while concurrently decreasing the nonspecific binding of the nonimmunoglobulin fraction. Any conventional purification procedure may be employed to purify the specific binding protein to remove nonessential fractions. Such procedures preferably result in the removal of serum albumin; $\alpha$-1 and $\alpha$-2 from the remaining mixed serum globulins. Removal of beta-globulin fraction is not required and if removed, may reduce the quantitative amount of specific binding protein present in the original sample.

Representative purification procedures include precipitation of the immunoglobulins with ammonium sulfate by conventional precipitation methods (see for example U.S. Patent 4,069,352) or removal of unwanted material using solubilizing organic solvents such as ethoxyacridine. These latter techniques involve selective solubilization of the specific binding protein and precipitation of the remaining nonessential fractions. Once recovered, the purified specific binding protein is used or stored until ready for use.

It has been found that when the specific binding protein (purified or nonpurified) is reacted with an aldehyde and inactivated in polypropylene tubes in the presence of a reducing agent, loss of antibody inhibitory activity is prevented.

While the exact mechanism for this effect is not known, it is believed to result from the prevention of oxidation of the specific binding protein by the presence of the reducing agent. It is known that proteins in neutral or alkaline pH can form disulfide bridges by in vitro reaction with dissolved oxygen. Haschemeyer, R.H. and A.E.V. Haschemeyer. 1973. "Primary structure of proteins" Proteins. Ed. John Wiley and Sons, Inc. p. 66. These bonds have the effect of altering the tertiary structure of the protein/antibody and hence can alter the enzymatic or immunoreactivity of the molecule. It is believed that the reducing agent has the chemical ability to prevent the formation of intramolecular sulfide bonds by rendering the sulfur group in a reduced sulfhydryl form, i.e., -SH. The specific binding protein/antibody is then stabilized in terms of immunoreactivity in the respective coating solution.

When coated on an insoluble plastic support, the specific binding protein generally exhibits greater binding of the labeled analyte than when the reducing agent is not incorporated. Immunoreactive stability of the specific binding protein, once bound to the solid phase surface, has been found to be quite good for long periods of time, even up to several months at 25°C.

The order in which the specific binding protein is contacted with the aldehyde compound and the reducing agent is not critical. It is only necessary that the specific binding protein be in contact with both the aldehyde compound and reducing agent prior to contact with the insoluble support. Contacting the insoluble support with either the antibody, aldehyde compound, or reducing agent prior to coating the supporting does not result in any added benefit by reducing loss of analyte inhibitory activity.

The reactions of the specific binding protein and aldehyde compound occurs over a narrow range of acidic and basic conditions. In order to ensure an optimum reaction, use of a buffer to control pH during the reaction has been found preferable. Acceptable material is obtained when buffer is employed in suitable amounts to control pH between 6.0 and 7.8 and preferably 7.0 and 7.5, Any suitable buffer may be selected for this purpose which does not affect the protein properties. Without being limited hereto, illustrative buffers include sodium carbonate, potassium carbonate, sodium or potassium phosphate (monobasic and/or dibasic), sodium chloride in a suitable buffer base and potassium chloride in a suitable buffer, and mixtures thereof.

Once the components are mixed they are allowed to react for a sufficient time at temperatures below about 37°C to form the aldehyde-analyte conjugate. Temperatures should be maintained between 4°C and 37°C and preferably between 20°C and 30°C for optimum efficiency. The reaction time may vary greatly from a few minutes to several hours and preferably from 5 to 60 minutes.

The aldehyde compounds used in the invention may be selected from a wide variety of monofunctional and bifunctional reagents which are water-soluble. Exemplary water-soluble monofunctional material may be represented by the formula $OCH-R_1$ wherein $R_1$ hydrogen, an alkyl group, and an aryl group. Representative compounds include formaldehyde, acetaldehyde, propionaldehyde, n-butylaldehyde and benzaldehyde, and mixtures thereof, with formaldehyde being preferred.

Exemplary water-soluble bifunctional material may be represented by the formula $OHC-(R_2)n-CHO$, wherein R may be $-(CH_2)-$, and wherein n is an integer from 2 to 10; such as an alkyl group, for example, glutaraldehyde; monocyclic alkyl group, for example, cyclopentyl or cyclohexyl; monocyclic aryl group, for example, phenyl; bicyclic aryl group, for example, naphthyl; substituted monocyclic alkane group, or substituted monocyclic aryl group, for example, tolyl and mixtures thereof. The preferred bifunctional aldehyde is glutaraldehyde.

The thus obtained aldehyde reacted specific binding protein is then brought into contact with an insoluble support and permitted to bond to the support in the presence of the reducing agent.

The reducing agents used in the invention may be selected from a wide variety of reagents which are reactive with disulfides to enable reduction of the sulfur moiety to the sulfhydryl form. Illustrative reducing agents include 1,4-dithiothreitol, 2-mercaptoethanol, thioglycollate, sodium borohydride, cysteine and mixtures thereof. The preferred material is 1,4-dithiothreitol because it is quite reactive in preventing the reoxidation of the sulfhydryl groups in the absence of blocking agents, and it enables retention of analyte activity over prolonged storage conditions.

The amount of reducing agent used is dependent upon the particular binding protein employed and the amount of sulfur containing amino acids, namely cysteine, present in the structure. In general, amounts of about 0.1milliMolar to about 2.0milliMolar, and preferably about 0.2milliMolar to about 0.8milliMolar have been found effective .

In a preferred embodiment, the aldehyde-specific binding protein complex is then brought into contact with an insoluble support in the presence of a reducing agent and permitted to bond to the internal surface of the support. This reaction can be obtained by incubation of the reaction product in the presence of the insoluble support under suitable temperature in the essential presence of a buffered solution. The temperature may vary widely depending upon the reaction time and coating efficiency desired and is preferably performed at temperatures from about 4°C to about 30°C. Buffering is essential to achieve maximum coating efficiency and is performed at pH values below 8.0, preferably between about 6.0 and about 7.5 and most preferably between about 7.0 and about 7.5. The pH value should be maintained during the entire coating period. Illustrative buffers include sodium carbonate, potassium carbonate, sodium phosphate (monobasic and dibasic), potassium phosphate (monobasic and dibasic), sodium chloride in a suitable buffer and potassium chloride in a suitable buffer, and mixtures thereof. The incubation time may vary widely depending on the degree of binding desired. Residence time of from 3 hours to 24 hours and preferably 10 hours to 20 hours have been found suitable to achieve % bindings greater than 40%.

Upon completion of incubation, the excess solution is decanted and the solid support preferably rinsed with distilled water to remove unbound components.

The resulting aldehyde-reducing agent-specific bonding protein reactant enhibits enhanced coating efficiency and long term storage stability.

Suitable solid support surfaces are formed from water-insoluble polymeric material capable of absorbing the specific binding protein. Exemplary materials include hydrocarbon polymers such as polystyrene, polyethylene, polypropylene and polybutylene. Other suitable organic polymers include silastic rubber, polyesters, polyamides, cellulose and cellulosic derivatives, acrylates, methacrylates, and vinyl polymers such as vinyl chloride, and polyvinyl fluoride. Copolymers such as graft copolymers of polystyrene are also useful.

A particularly effective form for the solid support surface is a test tube, bead, fin, well or microtitre plate. After completion of the contacting-absorption reaction, unbound protein and solution are removed from the insoluble support and the support surface is allowed to dry. Drying may be achieved by conventional techniques such as air drying or oven drying overnight at ambient temperatures up to 50°C. The resulting immunoassay tube has long term stability and excellent reproducibility. Storage may be undertaken at suitable temperatures such as from about 4°C to about 30°C.

Because the solid phase immunoassay system prepared by this process demonstrates a high degree of specific binding protein absorption without apparent loss by desorption when in use, this assay is a simple, rapid and practical means to provide an accurate analysis of small amounts of sample substances and is well suited for quantitative and qualitative determinations.

This invention encompasses a means for assaying for a specific analyte, such as an antigen, hapten or antibody in which a solid support surface is provided with a conjugate specific binding protein by the procedure of this invention. The insoluble support is then treated by conventional procedures to determine the quantitative and/or qualitative amount of material to be analyzed. For example, an unlabeled specimen analyte and a labeled specific analyte may contact the surface of the solid support of this invention to permit competitive reaction, followed by separation of bound and unbound material and measurement of the labeled component on the surface or in the removed solution to calculate the amount of specimen analyte present.

The labeled material used in the immunoassay of this invention may be any convention label, such as a radioactive isotope, an enzyme or a fluormetric material. When the analyte is labeled with an enzyme as described, for example, by Wilheimus in U.S. Patent 3,791,932, the amount of labeled analyte can be determined by measuring enzymatic activity of the tube. When the analyte is labeled with a fluorescent material the amount of labeled antigen can be determined by measuring fluorescense as described by U.S. Patent 3,789,116 to Kay. However the labeling is accomplished, the labeled material serves as a tracer.

A particularly preferred method involves labeling the specific binding protein with a radioactive isotope in a conventional manner. Suitable isotopes include $I_{125}$, $I_{131}$, $C_{14}$ or $H^3$. A particularly suitable isotope is the radioactive isotope of iodine, $I_{125}$, since labeling with this isotope is simple and many hospital laboratories have the equipment necessary to measure this material.

This invention also contemplates a kit for assaying a specimen for a specific analyte which may contain all or some of the materials including the assay tubes and instructions. Preferably the kit will contain the insoluble support with its surface coated with a specific binding protein according to the process of this invention and a labeled analyte.

The present invention is further illustrated by the following examples. All parts and percentages in the examples as well as in the specification and claims are by weight unless otherwise specified.

Example 1

Inventive Run 1 and Comparative Run A

Purified rabbit antiserum for L-3,5,3-triiodothyronine ($T_3$) is diluted 1 : 15,000 in a 0.5molar phosphate buffer having a pH of 7.5. Glutaldehyde is added to a final concentration of 5milliMolar (mM) and reacted for 20 minutes at room temperature. At the end of the reaction time 1,4-dithiothreitol (DTT) is added to a final concentration of 0.6 mM in Inventive Run 1 but not in comparative Run A. The solution is allowed to mix and one milliliter is then dispersed into polypropylene tubes, incubated overnight at 24°C, decanted, washed one time with a tris-gelatin buffer having a pH of 7.4 and allowed to air dry at 25°C.

A comparative analysis of tubes made in the presence and absence of DTT is presented in Figure 1. The results show the enhanced coating efficiency achieved when the reducing agent is present during the coating procedure. The results indicate that tubes prepared with the incorporation of DDT result in a superior solid phase immunoassay for the determination of $T_3$ uptake.

Example 2

Inventive Run 2 and Comparative Run B

Rabbit digoxin antiserum is purified with ethoxyacrydine (0.04%) and the soluble portion is dialyzed against dilute phosphate buffer. The purified antiserum is diluted 1 : 15,000 in a buffer containing 0.45m NaCl + 0.01 $mPO_4$ and reacted with glutaraldehyde at a 5.0 mM concentration for 20 minutes. 1,4-dithiothreitol is then added to a concentration of 0.6 mM in Inventive Run 2 but not in Comparative Run B. The solution is then mixed and 1.0 ml is added to polypropylene tubes and incubated O/N at 25°C. Tubes are decanted, washed once with a tris-gelatin buffered solution at pH 7.4, and then air dried. Figure II shows that binding of the digoxin antiserum is significantly increased over tubes made without the use of the 1,4-dithiothreitol.

Example 3

Inventive Runs 3 to 10 and Comparative Runs C to E

This example demonstrates use of the solid phase immunoassay of this invention in the determination of a specific analyte.

To determine the quantitative amount of analyte present in a sample specimen, 10 microliters of the specimen is placed into a previously prepared antibody analyte coated tube, which tube is prepared by the procedure of Example 1 and contained the ingredients recited in the Table. All ingredients were added in the order recited. Subsequently, a 1.0 milliliter sample of a radioactively labeled complementary antigen using an $I^{125}$ tracer in a buffer at pH 7.0, is added to the antibody coated tube. The reactants are incubated for 45 minutes at 24°C. The reactants are decanted and the percent bound fraction determined by a gamma counter which measures the $I^{125}$ activity and the percent bound of the specimen analyte computed from a dose response curve using known standards.

The utility of the invention for accurate measurement of $T_3$ in patients sera was demonstrated using sera having high, low and normal $T_3$ levels. The results are tabulated in Table 1 and are shown as percent uptake.

The results indicate the presence of a synergistic effect when both the aldehyde and reducing agent are present during the bonding reaction. The results also indicate that the order of addition of the aldehyde and reducing agent are not critical.

Table I

| | Patient Sample No. | Immunoassay Components: Antibody phosphate buffer/GLU | Antibody phosphate buffer/DTT | Antibody phosphate buffer GLU/DTT | Antibody phosphate buffer DTT/GLU |
|---|---|---|---|---|---|
| Standard sera* | | 19.8 | 16.6 | 25.2 | 26.1 |
| Low uptake control | C | 15.5 | 13.3 | 20.4 | 20.6 |
| Normal uptake control | D | 18.8 | 16.0 | 24.8 | 24.3 |
| High uptake control | E | 21.6 | 15.8 | 28.6 | 28.6 |
| Patient sera | 3 | 15.8 | 13.2 | 20.4 | 21.6 |
| Patient sera | 4 | 15.2 | 13.6 | 20.0 | 20.7 |
| Patient sera | 5 | 18.9 | 15.7 | 24.3 | 24.7 |
| Patient sera | 6 | 18.2 | 15.1 | 24.1 | 24.1 |
| Patient sera | 7 | 20.8 | 16.6 | 26.3 | N/A |
| Patient sera | 8 | 20.4 | 17.6 | 26.5 | 26.4 |
| Patient sera | 9 | 22.0 | 16.1 | 29.6 | 28.8 |
| Patient sera | 10 | 12.0 | 10.0 | 15.9 | 16.4 |

GLU = glutaraldehyde

DTT = 1,4-dithiothreitol

* Represents artificially prepared pool of normal human serum used as a reference point.

CLAIMS:

1. An insolubilized stable specific binding protein which is prepared by reacting a specific binding protein with an aldehyde compound and a reducing agent.

2. An insolubilized stable specific binding protein according to Claim 1, wherein the specific binding protein is a purified or non-purified immunoglobulin.

3. An insolubilized stable specific binding protein according to Claim 1 or 2, wherein the specific binding protein is chosen from:

an antibody, preferably an antibody containing immunoglobulin or immunoglobulin and beta-globulin;

an antigen; and

a hapten.

4. An insolubilized stable specific binding protein according to Claim 1, 2 or 3, wherein the aldehyde compound is chosen from one or more of the following:

a monofunctional aldehyde, preferably having the formula $R_1$-CHO wherein $R_1$ is a hydrogen atom, an alkyl group, or an aryl group, more preferably being formaldehyde; and

a bifunctional aldehyde, preferably having the formula OCH-$R_2$-CHO wherein $R_2$ is an alkylene group, a monocyclic alkylene group, a monocyclic arylene gorup, a bicyclic arylene group, a substituted monocyclic alkylene group, a substituted monocyclic arylene group, each of which has up to 10 carbon atoms, more preferably being glutaraldehyde.

5. A process for preparing a solid phase immunoassay, which process comprises:

effecting the reaction of a specific binding protein with an aldehyde compound and a reducing agent as specified in any preceding claim;

contacting the surface of an insoluble support with the reacted specific binding protein, thereby bonding the reacted specific binding protein to the surface; and

removing any remaining unbound protein from the insoluble support.

6. A process according to Claim 5, wherein the reaction of the specific binding protein with the aldehyde is performed in the presence of a buffer solution having a pH in the range from 6.0 to 7.8.

7. A process according to Claim 5 or 6, wherein the reacted specific binding protein is coated on the surface of an insoluble support in the presence of the reducing agent which is chosen from 1,4-dithiothreitol, 2-mercaptoethanol, thioglycollate, sodium borohydride, cysteine and mixtures thereof.

8. A process according to Claim 5, 6 or 7, wherein the reducing agent is present in an amount of from 0.1mM to 2.0mM.

9. A process according to Claim 6 or to Claim 7 or 8, when appendant to Claim 6, wherein the buffer is chosen from: sodium carbonate, potassium carbonate, sodium phosphate, sodium chloride, potassium chloride, and mixtures thereof.

10. A method of assaying a specimen for a specific component of the specimen, which method comprises:

reacting together a sample specific unlabelled component and a specific labelled component equivalent to the unlabelled component, preferably in the presence of a buffer, with a specific binding protein bound to an insoluble support; which specific binding protein is complementary to the specific components;

incubating the reactants;

removing the unbound reactants; and measuring the bound or unbound reactants and comparing the measurement with a standard curve; wherein the specific binding protein is bound to an insoluble support in the presence of an aldehyde compound and a reducing agent.

11. A process according to Claim 9 wherein the labelled component is radioactively labelled, enzyme labelled or fluorescently labelled.

12. An immunoassay kit containing an insolubilized stable specific binding protein as claimed in any one of Claims 1 to 4.

FIG.1

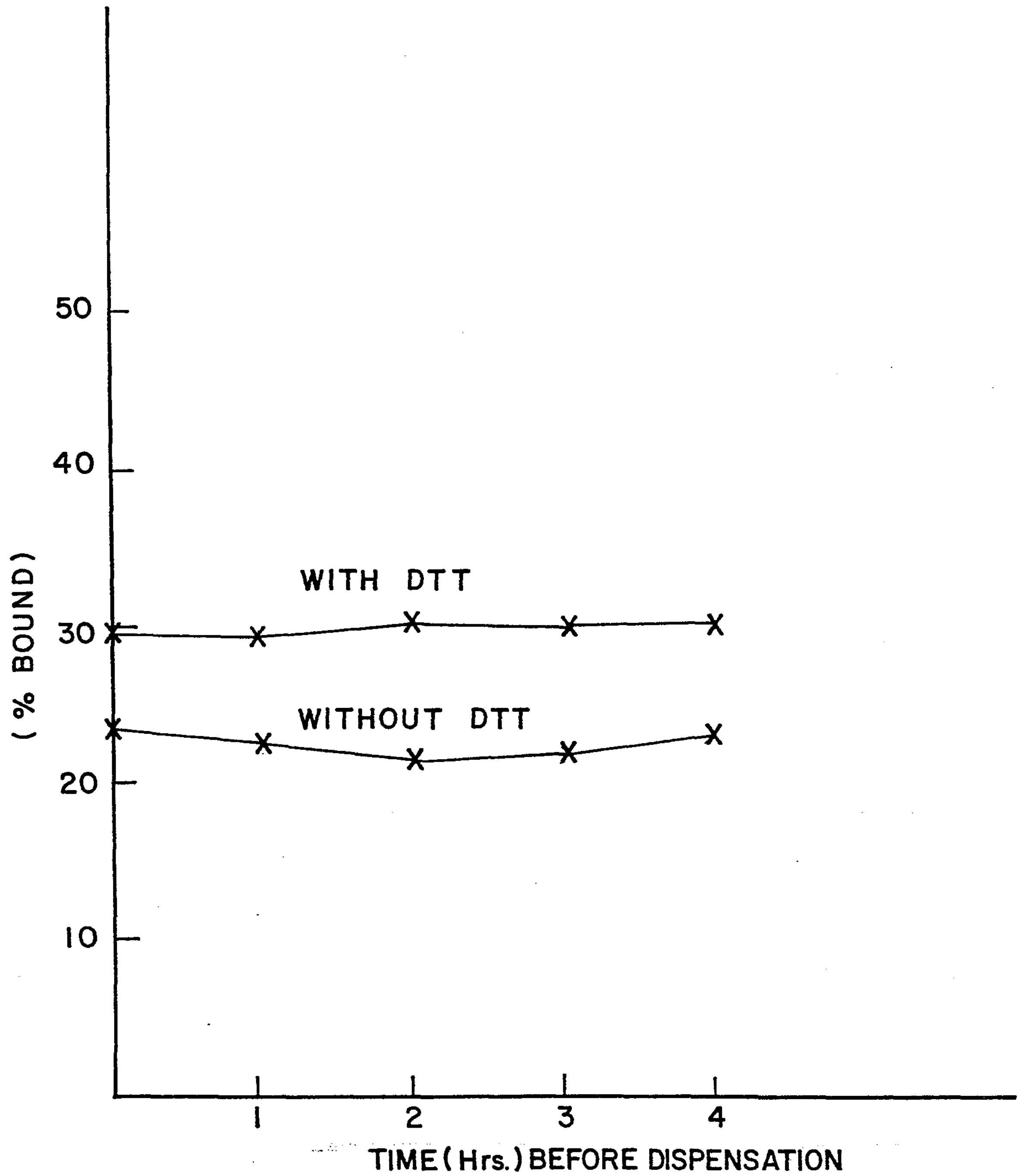
50
40
30
20
10
(% BOUND)
WITH DTT
WITHOUT DTT
1
2
3
4
TIME (Hrs.) BEFORE DISPENSATION

FIG. 2

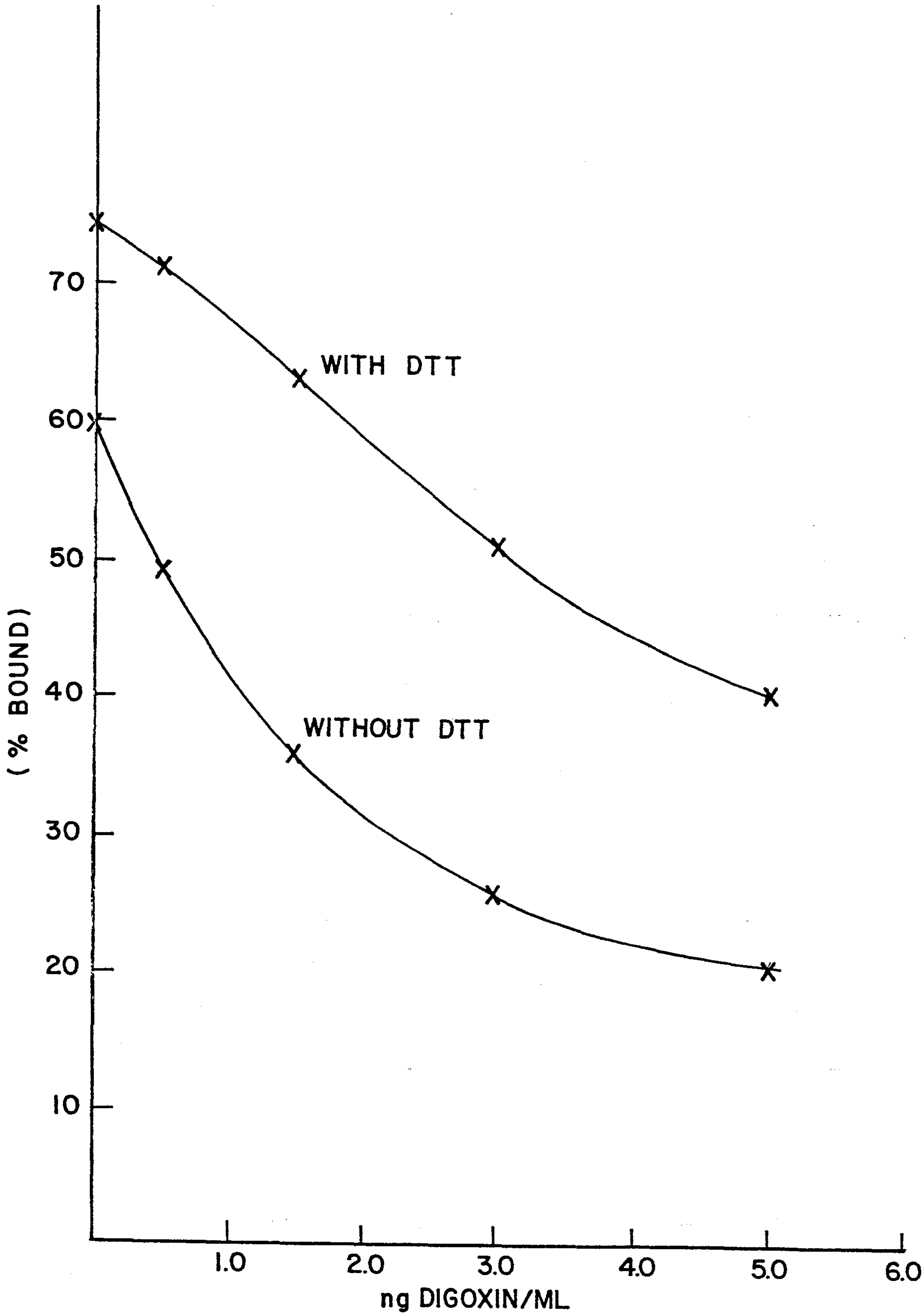
(% BOUND)
70
60
50
40
30
20
10
WITH DTT
WITHOUT DTT
1.0
2.0
3.0
4.0
5.0
6.0
ng DIGOXIN/ML